# EUROPÄISCHE PATENTANMELDUNG

(11) **EP 1 489 407 A1**
(43) Veröffentlichungstag der Anmeldung: **22.12.2004**
(21) Anmeldenummer: 04013399.3
(22) Anmeldetag: 07.06.2004
(51) Int. Cl.: G01N 27/04, G01N 27/12, C12Q 1/68, G01N 33/58

(54) **Verfahren und Vorrichtung zur Leitfähigkeitsmessung von Analyten mit elektrisch aktiven Markern**

(30) Priorität: 20.06.2003 DE 10327683
(71) Anmelder: Bayer Technology Services GmbH, 51368 Leverkusen (DE)
(72) Erfinder: Diessel, Edgar, Dr., 51061 Köln (DE); Burmeister, Jens, Dr., 51061 Köln (DE)

(57) **Zusammenfassung**

Die Erfindung betrifft ein Verfahren zum Nachweis eines oder mehrerer Analyten und eine Vorrichtung mit der das erfindungsgemäße Verfahren durchgeführt werden kann durch eine Erkennungsreaktion, unter Verwendung einer Vorrichtung mit einem mit Elektrolyten gefüllten Elektrolytraum (6) aufweisend mindestens eine Messelektrode (2, 3) mit gegebenenfalls einem, an die Messelektrode (2, 3) angrenzenden isolierenden Bereich (1) (Isolierschicht) und Erkennungsmolekülen, die auf der Messelektrode (2, 3) und/oder auf dem an die Messelektrode/n (2, 3) angrenzenden isolierenden Bereich immobilisiert sind und einer Gegenelektrode (5),
A) in Kontaktbringen von mit elektrisch aktiven Markierungseinheiten markierten Analyten mit den Erkennungsmolekülen, wobei die elektrisch aktiven Markierungseinheiten entweder vor dem Kontakt der Analyten mit Erkennungsmolekülen an die Analyten gebunden worden sind oder nach dem Kontakt der Analyten mit der biofunktionellen Oberfläche an die Analyten gebunden werden,
B) Applikation einer reaktiven Verstärkungslösung in den Elektrolytraum zur Verstärkung/Erzeugung der elektrischen Leitfähigkeit der elektrisch aktiven Markierungseinheiten,
C) Messung der elektrischen Leitfähigkeit bzw. des Widerstands zwischen der oder den Messelektrode/n und der Gegenelektrode in Abhängigkeit von der Entwicklungszeit,
D) Analyse des zeitl. Verlaufs der Leitfähigkeit bzw. des Widerstands nach C).

## Beschreibung

Die Erfindung betrifft ein Verfahren und eine Vorrichtung zum quantitativen elektrischen Nachweis von Analyten in einer Probe. Hierbei handelt es sich vorzugsweise um die spezifische Detektion eines biologisch relevanten Moleküls in einem wässrigen Medium. Ein derartiges Sensorprinzip bzw. ein solcher Sensor hat ein großes Anwendungsgebiet z.B. in der Umweltanalytik, dem Nahrungsmittelbereich, der Human- und Veterinärdiagnostik, dem Pflanzenschutz und in der biochemischen bzw. pharmakologischen Forschung.

Für derartige diagnostische Anwendungen sind Bio- und Chemosensoren bekannt, die eine biofunktionelle Oberfläche und einen physikalischen Signalwandler aufweisen. Unter einer biofunktionellen Oberfläche ist dabei eine Oberfläche zu verstehen, an die biologische, chemische oder biochemische Erkennungselemente gebunden sind.

An biofunktionelle Oberflächen werden biologische, chemische oder biochemische Erkennungselemente wie DNA, RNA, Aptamere, Rezeptoren gebunden, an die ein Analyt beim Nachweis mittels einer Erkennungsreaktion spezifisch bindet.

Beispiele für Erkennungsreaktionen sind die Bindung von Liganden an Komplexe, die Komplexierung von Ionen, die Bindung von Liganden an (biologische) Rezeptoren, Membranrezeptoren oder Ionenkanäle, von Antigenen oder Haptenen an Antikörper (Immunoassays), von Substraten an Enzyme, von DNA oder RNA an bestimmte Proteine, von Aptameren oder Spiegelmeren an ihre Targets, die Hybridisierung von DNA/RNA/PNA oder anderen Nukleinsäure-Analoga (DNA-Assays) oder die Prozessierung von Substraten durch Enzyme.

Beispiele für nachzuweisende Analyten sind DNA, RNA, PNA, Nukleinsäure-Analoga, Enzymsubstrate, Peptide, Proteine, potentielle Wirkstoffe; Medikamente, Zellen, Viren.

Beispiele für Erkennungselemente, an die die nachzuweisenden Analyten binden, sind DNA, RNA, PNA, Nukleinsäure-Analoga, Aptamere, Spiegelmere, Peptide, Proteine, Komplexbildner für Metalle/Metallionen, Cyclodextrine, Kronenether, Antikörper oder deren Fragmente, Anticaline, Enzyme, Rezeptoren, Membranrezeptoren, Ionenkanäle, Zelladhäsionsproteine, Ganglioside, Mono- oder Oligosaccharide.

Werden an die Oberfläche des Signalwandlers verschiedene Erkennungselemente räumlich voneinander getrennt gebunden, so kann eine große Zahl von Erkennungsreaktionen mit einer zu untersuchenden Probe zeitgleich durchgeführt werden. Verwirklicht ist dies z.B. bei sogenannten DNA-Arrays, bei denen verschiedene DNA-Sequenzen (z.B. Oligonukleotide oder cDNAs) auf einem festen Träger (z.B. Glas) immobilisiert sind. Solche DNA-Arrays werden in der Regel mit optischen aber auch mit elektrischen Methoden ausgelesen und finden Anwendung in der Expressions-Profilierung, der Sequenzierung, dem Nachweis viraler oder bakterieller Nukleinsäuren oder der Genotypisierung.

Die Detektion der Erkennungsreaktion bei Bio- oder Chemosensoren kann durch Anwendung optischer, elektrischer bzw. elektrochemischer, mechanischer oder magnetischer Signalwandlungsverfahren erfolgen.

Insbesondere die am weitesten fortgeschrittenen beschriebenen optischen Verfahren verfügen zwar über hohe Empfindlichkeiten lassen sich aber aufgrund des komplexen Aufbaus aus Lichtquelle, Sensor und Lichtdetektor in der Regel nur beschränkt miniaturisieren und sind daher den elektrischen Methoden in Bezug auf Herstellungskosten unterlegen.

Aus diesen Gründen kommt der Entwicklung von elektrischen Sensoren eine wachsende Bedeutung zu. Insbesondere führt der Einsatz von Mikrostrukturierungstechnik aus der Halbleitertechnologie zu miniaturisierten Formaten, die hohe Empfindlichkeiten bei geringen Herstellungskosten versprechen. Insbesondere sind Methoden vorteilhaft, die Markierungseinheiten für den Analyten benutzen, deren Eigenschaften sich signifikant von denen der Bestandteile der zu analysierenden Probe unterscheiden. Hierzu bieten sich z.B. metallische Nanopartikel als Markierungseinheiten an.

Im Rahmen von Gleichstrommessungen besitzen bestimmte elektrische Biosensoren mit metallischen Nanopartikeln das Potenzial für eine außerordentlich hohe Empfindlichkeit bis in den Einzelmolekülbereich. Dieses Potenzial erschließt sich insbesondere durch autometallografische Abscheidung. Bei diesem sog. Autometallografie-Prozess, der aus der Fotografie und der Elektronenmikroskopie bekannt ist, wirken die Nanopartikel oder Kolloide als Katalysatoren für den Elektronenübertrag von einem Reduktionsmittel auf ein Au- oder Ag-Ion, das sich in Form eines Ag oder Au-Salzes mit dem Reduktionsmittel wie z.B. Hydrochinon in der Verstärkungslösung befindet. Nach erfolgter Reduktion fällt das Ion auf dem Kolloid als Metall aus. Als elektrischer Signalwandler werden hierfür Elektrodenpaare gewählt, die durch einen Isolator voneinander getrennt sind. Mit Nanopartikeln markierte Analytmoleküle bilden mit autometallographischer Verstärkung eine leitfähige Brücke zwischen den Elektroden, die durch eine Gleichstrom-Widerstandsmessung detektiert wird. Die grundlegende Technik hierzu ist beschrieben in: US-A- 5,284,748. Weitere Offenbarungen bezüglich der Anwendung auf die DNA-Selektion finden sich in WO 99/57550-A2 und in WO 01/00876-A2. Die Detektion von Nukleinsäuren durch Gleichstrom-Widerstandsmessung wurde demonstriert (vgl. Möller et al., Langmuir 17, 5426 (2001)). Als weitere Ausbaustufe dieser Methode wird die Diskriminierung von Punktmutationen (Single Nucleotide Polymorphisms (SNPs)) in (Park et al., Science 295, 1503 (2002)) beschrieben. Quantifizierung mit dieser Methode ist zwar möglich jedoch sehr aufwendig, da eine Kette von Verstärkungszyklen mit anschließenden punktuellen Messungen durchlaufen werden muss.

Nachteil der bekannten Verfahren sind komplizierte Folgen von Aufbringungs-, Entwicklungs-, Wasch- und Trockenschritten, wobei evtl. Entwicklungs-, Wasch- und Trockenschritte wiederholt werden müssen.

Ein Konzept zur praktikableren Lösung für eine Quantifizierung wird in WO 02/02810-A2 beschrieben. Hier wird der elektrisch leitfähige Niederschlag auf den Markierungseinheiten während der Abscheidungsreaktion mittels Leitfähigkeitsmessungen zwischen zwei paarweise angeordneten Mikroelektroden detektiert. Die Leitfähigkeit zwischen den Elektroden wird durch das Ausbilden einer metallischen Brücke aufgrund der Erkennungsreaktion verändert.

Nachteil dieses Verfahrens ist, dass für jede dieser Anforderungen pro Messfläche zwei Mikrostrukturelektroden erforderlich sind, die benachbart sind und daher Störungen der Leitfähigkeitsmessung durch unbeabsichtigte Kurzschlüsse die Messung auf DNA verfälschen können.

Alternativ zum Ausbilden einer metallischen Brücke zwischen zwei Elektroden sind Verfahren bekannt, die auf einer Elektrode gebundenen Analyten mittels autometallografisch verstärkten Metallkolloide elektrochemisch nachweisen (Cai et al., Analytica Chimica Acta 469, 165-172 (2002)).

Nachteil dieses Verfahrens ist die vergleichsweise kleine Messfläche, die zu einem schlechten Signal-Rauschverhältnis führt. Ferner sind wiederum gegebenenfalls verfälschende aufwendige Wasch- und Trockenschritte zur Vorbereitung der Messung notwendig.

Der zeitliche Verlauf des Wachstumsprozesses kann hiermit ebenfalls nicht verfolgt werden.

Der Erfindung liegt die Aufgabe zugrunde, eine hochempfindliche elektrische Messvorrichtung und eine Messmethode zum Nachweis von Analyten mittels Erkennungsreaktionen zu entwickeln, der die Nachteile des Standes der Technik vermeidet.

Die Lösung der erfindungsgemäßen Aufgabe besteht in einem
Verfahren zum Nachweis eines oder mehrerer Analyten durch eine Erkennungsreaktion, unter Verwendung
einer Vorrichtung mit einem mit Elektrolyten gefüllten Elektrolytraum aufweisend
mindestens eine Messelektrode mit gegebenenfalls einem, an die Messelektrode angrenzenden isolierenden Bereich (Isolierschicht)
Erkennungsmolekülen, die auf der Messelektrode und/oder auf dem an die Messelektrode/n angrenzenden isolierenden Bereich immobilisiert sind und einer Gegenelektrode,

A) in Kontaktbringen von mit elektrisch aktiven Markierungseinheiten markierten Analyten mit den Erkennungsmolekülen, wobei die elektrisch aktiven Markierungseinheiten entweder vor dem Kontakt der Analyten mit Erkennungsmolekülen an die Analyten gebunden worden sind oder nach dem Kontakt der Analyten mit der biofunktionellen Oberfläche an die Analyten gebunden werden,
B) Applikation einer reaktiven Verstärkungslösung in den Elektrolyträum zur Verstärkung/Erzeugung der elektrischen Leitfähigkeit der elektrisch aktiven Markierungseinheiten,
C) Messung der elektrischen Leitfähigkeit bzw. des Widerstands zwischen der oder den Messelektrode/n und der Gegenelektrode in Abhängigkeit von der Entwicklungszeit,
D) Analyse des zeitlichen Verlaufs der Leitfähigkeit bzw. des Widerstands nach C).

Der Kern der Erfindung betrifft die Onlinemessung des elektrischen Widerstands oder der Leitfähigkeit zwischen der Messelektrode und der Gegenelektrode der Messvorrichtung während sich eine geeignete Verstärkungslösung auf der Messvorrichtung befindet. Die Messelektrode zusammen mit dem angrenzenden isolierenden Substratbereich werden im folgenden Messfläche genannt. Die Größe der Messfläche wird durch die Fläche des zugehörigen Bereichs immobilisierter Erkennungsmoleküle definiert. Es wurde gefunden, dass während des Verstärkungsprozesses eine signifikante Veränderung des Widerstands oder der Leitfähigkeit zwischen Messelektrode und Gegenelektrode gemessen werden kann. Eine Deutung für den Widerstandsabfall besteht darin, dass zu diesem Zeitpunkt die leitfähigen Markierungseinheiten, die sich auf dem Isolator befinden, eine leitfähige Schicht ausbilden und mit der Messelektrode leitfähig verknüpft werden. Hiermit wird die Elektrodenfläche stark vergrößert. Die Messelektrode kann hierbei mit Erkennungs-DNA belegt sein. Dies ist jedoch nicht nötig, wenn während des Ausbildens der leitfähigen Schicht durch die räumliche Nähe der Markierungseinheiten auf dem Isolator zur Messelektrode die Markierungseinheiten mit der Elektrode elektrisch leitend verknüpft werden.

Um die Elektrodenfläche über den Bereich der immobilisierten Erkennungsmoleküle hinaus durch den Verstärkungsprozess zu vergrößern, wird in einer bevorzugten Ausführungsform der Sensorvorrichtung in der Nähe der Messelektrode zusätzlich eine von der Messelektrode isolierte leitfähige Schicht einer bestimmten Fläche aufgebracht. Hierbei braucht diese Fläche nur partiell von dem Bereich der Probenmoleküle bedeckt zu sein. Durch die Erkennungsreaktion mit nachfolgender Verstärkung wird diese Fläche elektrisch leitend mit der Messelektrode verknüpft, womit die Leitfähigkeitserhöhung bzw. Widerstandserniedrigung zusätzlich verstärkt wird.

Zum Nachweis können alle bekannten Verfahren aus der Elektrochemie wie z.B. Cyclo-Voltammetrie angewendet werden. Diesbezüglich können in 3-Elektrodenanordnungen Potentiostatschaltungen zum Einsatz gebracht werden. Hierzu wird in einer bevorzugten Ausführung zusätzlich zu Mess- und Gegenelektrode eine Referenzelektrode in den Messaufbau integriert, wobei die Referenzelektrode gegenüber der Messelektrode auf konstanter Spannung gehalten wird. Aufgrund der Einfachheit wird in einer besonders bevorzugten Ausführungsform nur in einer 2-Punkt-Geometrie mit Mess- und Gegenelektrode gemessen. Hierzu kann entweder a) eine Spannung oder b) ein Strom zwischen der oder den Messelektroden und einer Gegenelektrode während des Verstärkungsprozesses angelegt werden, und entweder im Fall a) der Strom oder im Fall b) die Spannung zwischen den Elektroden während des Verstärkungsprozesses gemessen werden. Generell werden im Text mit der Bezeichnung von Widerstands- und Leitfähigkeitsmessungen im breitesten Sinne die vorgenannten Methoden sowohl in 2-Punkt als auch in 3-Punkt-Geometrie eingeschlossen.

Die Onlinemessung gestattet insbesondere eine Quantifizierung über die Analyse des zeitlichen Verlaufs des Widerstands oder der Leitfähigkeit. Je höher die Dichte der elektrisch aktiven Markierungseinheiten auf der Messfläche nach einer erfolgten Erkennungsreaktion ist, desto früher wird ein deutlicher Widerstandsabfall oder einer Leitfähigkeitszunahme erreicht. Dies spiegelt sich insbesondere durch ein Extremum in der 1. Ableitung, des Gradienten, im zeitlichen Verlauf wider. Es ergibt sich ein monotoner Zusammenhang zwischen der Zeitdauer bis zum Erreichen dieses Verhaltens und der Konzentration des Analytens, der zur Quantifizierung herangezogen werden kann. Zur Erhöhung der Genauigkeit der Quantifizierung kann die Kurvenform des zeitlichen Verlaufs der elektrischen Kenngröße mit einer geeigneten mathematischen Funktion angepasst werden. In diesem Fall wird aus den Kurvenparametern die Analytkonzentration bestimmt. Zur Quantifizierung eines Analytens werden die zugehörigen Zeitdauer bzw. Parameter aus der Kurvenanpassung mit denen aus mindestens einer Kontrollprobe mit bekannter Analytkonzentration verglichen.

Bevorzugt ist daher auch ein Verfahren, dadurch gekennzeichnet, dass die Konzentrationsangabe in einem quantitativen Assay bzw. der grundsätzliche Nachweis in einem qualitativen Assay über die Zeitdauer der Reaktion B) bis zum Erreichen eines signifikanten Gradienten im zeitlichen Verlaufs von Widerstand oder Leitfähigkeit mit Zeitdauer der Reaktion B) einer Referenzprobe verglichen und zur quantitativen oder qualitativen Analyse des Analyten verwendet wird.

Die Immobilisierung der Erkennungsmoleküle auf der Messfläche und/oder der angrenzenden Isolierschicht erfolgt insbesondere durch dem Fachmann grundsätzlich bekannte Methoden. Für DNA-Erkennungseinheiten ist diese Immobilisierung z.B. in S. L. Beaucage, Curr. Med. 8, 1213-1244 (2001) beschrieben. Für die Immobilisierung auf der Messfläche wird eine optimale Dichte von Erkennungseinheiten angestrebt, die bei hoher Flächendichte eine optimale Aktivität der Erkennungseinheit gewährleistet. Die Immobilisierung von Erkennungselementen wie Antikörpern kann kovalent oder nichtkovalent erfolgen. Beispielsweise können Avidin oder Streptavidin auf der Oberfläche physisorbiert oder nach geeigneter Biofunktionalisierung der Oberfläche kovalent immobilisiert werden. An die mit Avidin oder Streptavidin beschichtete Oberfläche können beispielsweise biotinylierte Antikörper spezifisch immobilisiert werden.

Bevorzugt sind Erkennungselemente für die Analyten an eine Messfläche mit biofunktioneller Oberfläche gebunden. Die Analyten gehen mit den Erkennungselementen eine Erkennungsreaktion ein. Der Analyt kann vor der Bindung an das Erkennungselement bereits mit einer elektrisch aktiven Markierungseinheit markiert sein oder er wird erst nach der Bindung an das Erkennungselement markiert, indem z.B. ein Bindungselement, das mit einer Markierungseinheit markiert ist, an den Komplex bestehend aus Erkennungselement und Molekül bindet.

Die Analyten können durch die Erkennungsreaktion auch indirekt nachgewiesen werden und müssen daher nicht notwendigerweise markiert werden. Bei der indirekten Detektion werden Analyten, die bereits vor Bindung an das Erkennungselement mit Markierungseinheiten markiert sind, in Kontakt mit der biofunktionellen Oberfläche gebracht. Gleichzeitig werden zusätzlich unmarkierte Analyten in Kontakt mit der biofunktionellen Oberfläche gebracht. Diese beiden Spezies kompetieren um die Bindung an die immobilisierten Erkennungselemente. Befinden sich in der Messlösung über der Messfläche keine unmarkierten Analyten, so werden alle Bindungsplätze an den Erkennungselementen von markierten Analyten besetzt und die Modifikation des Widerstands oder der Leitfähigkeit wird maximal. Für eine nichtverschwindende Konzentration von unmarkierten Analyten werden die Bindungsplätze an den Erkennungselementen entsprechend der jeweiligen Konzentrationen von unmarkierten Analyten und markierten Analyten anteilig besetzt, so dass die Modifikation des Widerstands im Vergleich zur verschwindenden Konzentration des unmarkierten Analytens kleiner ist. Die Kalibration dieses Mischsystems erfolgt durch die Messung von mindestens einer Probe, die nur den markierten Analyten mit einer bekannter Konzentration enthält.

Bevorzugt ist daher eine Variante des Verfahrens, dadurch gekennzeichnet, dass in Schritt A) eine quantitativ vorbestimmte Menge eines bekannten Analyten, der mit Markierungseinheiten versehen ist, gemischt wird mit der Probe eines unmarkierten bekannten Analyten und aus einem Vergleich der Analyse D) dieses Mischsystems mit der Analyse des reinen bekannten markierten Analyten die Konzentration des unmarkierten Analyten bestimmt wird.

Mit dem erfindungsgemäßen Verfahren lässt sich die Modifikation der Leitfähigkeit bzw. des Widerstands zwischen den Elektroden durch eine einzige Markierungseinheit pro Analytmolekül nachweisen. Um die Empfindlichkeit der Methode noch zusätzlich zu steigern, können Analytmoleküle mit mehreren elektrisch aktiven Markierungseinheiten versehen werden.

Nach dem bevorzugten Verfahren werden Analyten mit Markierungseinheiten markiert, die in geeigneter Weise elektrisch aktiv sind. Die elektrische Aktivität kann in der elektrischen Leitfähigkeit des für die Markierungseinheiten verwendeten Materials bestehen, die vorzugsweise im Bereich metallischer Leitfähigkeiten liegt. Außerdem können Monomere elektrisch leitfähiger Polymere als elektrisch aktive Elemente gewählt werden. Schließlich werden auch Enzyme als elektrisch aktive Elemente betrachtet, wenn sie eine Reaktion katalysieren, die zu leitfähigen Produkten führt wie z.B. leitfähige Polymere.

Als elektrisch aktive Markierungseinheiten können Nanopartikel, Metallkomplexe und/oder Cluster aus leitfähigen Materialien wie Au, Ag, Pt, Pd, Cu eingesetzt werden.

Die Größe der elektrisch aktiven Markierungseinheiten liegt bevorzugt im Bereich von 1 bis 100 nm, insbesondere bevorzugt im Bereich von 1 bis 30 nm, besonders bevorzugt im Bereich von 1 bis 2 nm. Die letztgenannte Größe wird z.B. durch Au-Cluster bestehend aus 50-150 Atomen realisiert. Die Größenangabe bezieht sich dabei auf den größten Durchmesser der Markierungseinheiten.

Weiterhin können nichtleitfähige Partikel mit leitfähiger Beschichtung oder nichtleitfähige Partikel mit metallischer Beschichtung als Markierungseinheiten eingesetzt werden. Nichtleitfähige Partikel können z.B. Polystyrolbeads sein.

Markierungseinheiten können bevorzugt auf leitfähigen Polymeren basieren z.B. Polyaniline, Polythiophene, insbesondere Polyethylendioxythiophen, Polyphenylene, Polyphenylenvinylen, Polythiophenvinylen, Polypyrrole.

Bevorzugt ist auch eine Variante des Verfahrens, dadurch gekennzeichnet, dass die elektrisch aktiven Markierungseinheiten auf Enzymen, bevorzugt HRP basieren, die durch die Umsetzung eines Substrats ausgewählt aus Anilin oder Ethylendioxythiophen elektrisch aktive Markierungseinheiten bilden.

Eine weitere Anwendung von HRP ist die Abscheidung eines Polymers, an das z.B. Nanopartikel oder alle oben beschriebenen Markierungseinheiten direkt oder indirekt über Biotin-Streptavidin, Biotin-Avidin oder Biotin-NeutrAvidin gebunden werden. Für den indirekten Fall ist das Polymer biotinyliert. Dieses Prinzip ist als Catalyzed Reporter Deposition (CARD) bekannt.

Besonders bevorzugt ist ein Verfahren, dadurch gekennzeichnet, dass die elektrisch aktive Markierungseinheit auf einem Enzym basiert, das die Bildung eines nichtleitfähigen Polymeren, insbesondere eines biotinylierten Polymeren katalysiert, das seinerseits direkt oder indirekt mit Nanopartikeln, Metallkomplexen oder Clustern auf Basis von Elementen aus der Reihe Au, Ag, Pt, Pd, Cu oder mit elektrisch leitfähigen Polymeren verbunden ist.

Geeignete Verstärkungslösungen sind abhängig von der Art der gewählten elektrisch aktiven Markierungseinheiten. Im Falle von Nanopartikeln, Metallkomplexen und/oder Clustern aus leitfähigen Materialien, nichtleitfähige Partikel mit metallischer Beschichtung werden besonders vorteilhaft autometallografische Verstärkungslösungen auf der Basis von Ag- oder Au-Salzen zur Signalverstärkung eingesetzt. Hierbei werden als Reduktionsmittel z.B. Hydrochinon oder Formaldehyd eingesetzt. Für Monomere elektrisch leitfähiger Polymere können Verstärkungslösungen aus Katalysatoren, Initiatoren und/oder weiteren Monomeren dieser Polymere bestehen, die zur Polymerisation benötigt werden. Für die Polymerisierung von Anilin kann als Katalysator z.B. HRP eingesetzt werden. Für Enzyme als elektrisch aktive Markierungseinheiten z.B. HRP können die Monomere eines elektrisch leitenden Polymers z.B. Anilin als Verstärkungslösung verwendet werden.

Verstärkungslösungen können oberhalb elektrischer Markierungseinheiten im Laufe des Verstärkungsprozesses ihre Konzentration ändern, so dass der Verstärkungsprozess in eine Sättigungsphase eintritt. Um den Verstärkungsprozess in diesem Fall fortzuführen, soll die Verstärkungslösung bevorzugt ausgetauscht werden. Dies kann durch Rühren oder durch kompletten Austausch der Flüssigkeit z.B. im Rahmen eines mikrofluidischen Flusssystems erfolgen. Der Austausch erfolgt vorzugsweise kontinuierlich.

Das erfindungsgemäße Verfahren kann z.B. für die Analyse von Peptiden, Proteinen oder Nukleinsäuren eingesetzt werden. Bevorzugt ist die in dem Verfahren angewendete Erkennungsreaktion A) ein Peptid- oder Proteinassay insbesondere ein Immunoassay oder ein Nukleinsäure-, insbesondere ein RNA- oder DNA-Assay, besonders bevorzugt ein SNP-Assay. DNA-Assays dienen bevorzugt zur Detektion von viraler DNA oder RNA oder von DNA bakterieller Spezies, sowie zur Expressions-Profilierung, zur Genotypisierung für die Diagnose von Erbkrankheiten oder für Pharmacogenomics (genetisch bedingte Wirkweise bzw. Nebenwirkungen von Pharmaka), Nutrigenomics (genetisch bedingte Wirkweise bzw. Nebenwirkungen von Nahrungsmitteln). Bei der Genotypisierung werden insbesondere Veränderungen von Genen festgestellt, die nur auf der Variation einer Base beruhen (single nucleotide polymorphism = SNP).

Die erfindungsgemäße Methode erlaubt die simultane Analyse einer Vielzahl von Analyten, indem für derartige Multiplex-Analysen eine entsprechende Vielzahl von Messflächen zur Verfügung gestellt wird. Hierbei wird pro Messfläche ein Analyt detektiert. Auf mehreren Messflächen können auch zur Mehrfachdetektion eines Analytens jeweils die gleichen Erkennungselemente immobilisiert sein. Einzelne Messflächen können z.B. zur Detektion von Referenzsubstanzen, die den Einfluss von z.B. Temperatur, Licht auf den Verstärkungsprozess charakterisieren, verwendet werden. Diese Referenzwerte können zur Normalisierung der Signale anderer Messflächen herangezogen werden. Bevorzugt ist daher auch ein Verfahren, dadurch gekennzeichnet, dass eine Vielzahl von Erkennungsreaktionen mit den Schritten A) bis D) parallel simultan auf einer Sensorvorrichtung durchgeführt werden, indem die Messvorrichtung mit einer Vielzahl von Messelektroden versehen wird, auf denen jeweils gleiche oder unterschiedliche Erkennungselemente angebracht sind. Für wissenschaftliche Anwendungen werden Multiplex-Assays mit Anzahlen > 1000 verwendet, während für diagnostische Anwendungen mit Zahlen 1 - 1000 gerechnet wird. Aufgrund der möglichen Vielzahl von Messflächen kann die Vorrichtung gleichzeitig zum Nachweis einer Vielzahl von Proteinen oder Peptide als Proteinarray oder Peptidarray bzw. für den Nachweis von Nukleinsäuren als Nukleinsäure-Array eingesetzt werden.

Insbesondere werden bis zu 1000 Erkennungsreaktionen simultan auf einer Sensorvorrichtung durchgeführt.

Die Detektion des Analyten wird insbesondere in wässrigem Medium durchgeführt. Bevorzugt als wässrige Medien sind Körperflüssigkeiten wie Blut, Speichel, Urin, Schweiß, interstitielle Flüssigkeit und Tränenflüssigkeit.

Gegenstand der Erfindung ist auch eine Vorrichtung zum Nachweis eines oder mehrerer Analyten durch eine Erkennungsreaktion, insbesondere nach dem beschriebenen Verfahren, wenigstens umfassend
- mindestens eine Messelektrode und mindestens einen angrenzenden isolierenden Bereich (Isolierschicht)
- Erkennungsmoleküle, die auf dem an die Elektrode/n angrenzenden isolierenden Bereich und optional auf der Elektrode/n immobilisiert sind, eine Gegenelektrode
- Analyten, die mit elektrisch aktiven Markierungseinheiten markiert sind gegebenenfalls eine Verstärkungslösung und
ein Messgerät zur zeitaufgelösten Erfassung der Leitfähigkeit bzw. des Widerstands zwischen der oder den Messelektrode/n und Gegenelektrode während des Verstärkungsprozesses.

In der erfindungsgemäßen Vorrichtung weisen die Messflächen, Erkennungselemente, Analyten, elektrisch aktiven Markierungseinheiten und Verstärkungslösungen, bevorzugt die zur Durchführung des Verfahrens oben beschriebenen Eigenschaften auf.

Die Bestimmung der Leitfähigkeit wird z.B. in einer 2- oder 3-Punktgeometrie durchgeführt. In beiden Anordnungen können die Gegenelektrode bzw. die Referenz- und Gegenelektrode zusammen mit der Messfläche auf einem gemeinsamen Substrat angebracht sein oder als separate Elektroden in einer Messzelle ausgeführt werden Dies ermöglicht eine kostensparende Herstellung der Messzelle.

Auf einem Substrat werden dem Verfahren gemäß ein oder vorteilhaft mehrere Messflächen aufgebracht. Dementsprechend werden auf den einzelnen Messflächen jeweils eine Spezies von Erkennungselementen immobilisiert. Unterschiedliche Messflächen können jeweils gleiche oder paarweise unterschiedliche Spezies von Erkennungselementen tragen.

Bevorzugt ist daher eine Vorrichtung, dadurch gekennzeichnet, dass die Oberfläche der Messvorrichtung eine Vielzahl von Messelektroden aufweist, auf denen jeweils gleiche oder unterschiedliche Erkennungsmoleküle angebracht sind.

Die Messelektroden können vorteilhaft durch Siebdrucktechniken mit Strukturbreiten zwischen ca. 100 µm und 1 mm realisiert werden. Optische Lithografiemethoden erlauben laterale Strukturgrößen von ca. 2 µm. Wesentlich kleinere laterale Dimensionen werden durch Elektronenstrahltechniken erzielt. Je kleiner die Messelektrode im Vergleich zum immobilisierten Bereich der Erkennungselemente ist, desto höher ist wahrscheinlich die erreichbare Empfindlichkeit. Mit Messflächen von z.B. 100 µm² lassen sich auf einem 100 mm² großen Chip 10⁶ Elemente unterbringen. Für diagnostische Anwendungen mit ca. 100 Analyten sind dagegen Messflächen von bis zu 1 mm² auf der gleichen Fläche mit vergleichsweise geringerem Aufwand realisierbar.

Diese Größenangaben haben lediglich Beispielcharakter und schließen andere Größen und Anzahlen nicht aus. Um viele Elektroden einzeln anzusteuern, werden Multiplexschaltkreise eingesetzt.

Die Größe der mit Erkennungsmolekülen belegten Messfläche beträgt bevorzugt 100 µm bis 1 mm.

Aufgrund der möglichen hohen Packungsdichte der Messflächen eignet sich die erfindungsgemäße Vorrichtung als Plattform für Nukleinsäure- und Proteinarrays.

Bevorzugt ist auch eine Ausführung der Vorrichtung, dadurch gekennzeichnet, dass die Sätze von Erkennungsmolekülen mit Messelektroden ein Nukleinsäure-Array, ein Peptid-Array oder ein Protein-Array bilden.

Um die Verstärkungslösung nicht unspezifisch durch elektrochemische Prozesse an den elektrischen Zuleitungen zu verarmen, werden vorteilhaft die Zuleitungen zu den Elektroden von der Verstärkungslösung isoliert. Dies kann z.B. durch die Abscheidung von SiO₂ auf die Zuleitungen realisiert werden.

Die Elektroden können planar oder in nichtplanaren Geometrien ausgeführt werden.

Bevorzugt ist auch eine Vorrichtung, dadurch gekennzeichnet, dass die Messelektroden auf einem Substrat planar nebeneinander ausgeführt sind.

In der planaren Ausführung befinden sich eine oder mehrere Messflächen seitlich nebeneinander auf einem Substrat. Analyt- und Verstärkungslösungen können über Mikrokanäle, die in das Substrat geätzt sind, den Messflächen zugeführt werden. Hierbei befinden sich die Messflächen z.B. auf dem Boden dieser Kanäle. Alternativ kann ein mit Mikrokanälen versehenes Bauteil als Deckel für ein planares Substrat eingesetzt werden.

Besonders vorteilhaft können mehrere Messflächen vertikal übereinander in Form von alternierenden Schichtstrukturen von Elektroden und Isolatorschichten ausgeführt werden, da hier Abscheidungsverfahren aus der Halbleitertechnik Anwendung finden können. Zusätzlich können diese Schichtenpakete mit parallel zur Flächennormalen dieser Schichten angebrachten Mikrokanälen versehen werden, durch die Analyt- und Verstärkungslösungen auf die Messflächen appliziert werden. Ein Multiplex-Assay wird insbesondere dadurch realisiert, dass ein Satz von Mikrokanälen in einer Schichtstruktur mit mindestens je einer Deck- und Basisschicht (elektrischer Isolator) und wenigstens einer Zwischenschicht aus alternierend nebeneinander angebrachten elektrisch leitenden Schichten (Messschichten) und Isolatorschichten angebracht wird. Die Kanäle sind nebeneinander angeordnet und durchtreten unabhängig voneinander elektrisch ansteuerbare Messschichten, wobei die einzelnen Kanäle mit unterschiedlichen Erkennungs-DNAs bestückt werden. Alternativ können in einem Schichtenpaket mehrere übereinander liegende Messflächen erzeugt werden, indem mehrere durch Isolatorschichten vertikal voneinander isolierte Schichten aus alternierend nebeneinander angebrachten leitenden Messschichten und Isolatorschichten von den Mikrokanälen durchdrungen werden. Bei elektrischer Kontaktierung der einzelnen leitenden Messschichten werden so in einzelnen Mikrokanälen übereinander angeordnete Paare von Elektroden und Isolatoren realisiert. Werden zwischen den Elektroden eines einzigen Mikrokanals der letztgenannten Anordnung selektiv verschiedene Erkennungs-DNAs immobilisiert, wird mit diesem Aufbau ein multiplexfähiger Mikrokanal für die Online-Widerstands- bzw. Leitfähigkeitsmessung realisiert. Die ortsaufgelöste DNA-Immobilisierung in einem Mikrokanal kann durch die elektrische Anziehung von Polystyrolbeads, auf deren Oberflächen Erkennungs-DNA - immobilisiert ist, zwischen zwei vorwählbaren Elektroden erfolgen, s. Velev et al., Langmuir 15, 3693 (1999).

Die Probenzuführung wird vorteilhaft über Mikrokanäle, die senkrecht zur Messfläche eine Höhe im Bereich zwischen 1 und 1000 µm, bevorzugt 1 - 50 µm besitzen, bewerkstelligt. Die Größenangaben sind durch die Diffusionskoeffizienten von Biomolekülen im Zusammenhang mit Inkubationszeiten im Sekunden- bis in den unteren Minutenbereich von 1-10 Minuten motiviert.

Bevorzugt ist auch eine Ausführungsvariante der Vorrichtung, die dadurch gekennzeichnet ist, dass der Elektrolytraum durch einen oder mehrere Kanäle gebildet wird, die eine Vielzahl von Messelektroden aufweisen und eine Höhe von maximal 100 µm über den Messelektroden aufweisen.

Um einer Verarmung der Verstärkungslösung induziert durch die elektrisch aktiven Markierungseinheiten vorzubeugen, wird die Verstärkungslösung bevorzugt durch die oben beschriebenen mikrofluidischen Kanäle schrittweise oder kontinuierlich ausgetauscht. Außerdem ist eine Vorrichtung vorzusehen, die den Flüssigkeitsaustausch treibt. Hierzu können Pumpen bzw. Spritzen verwendet werden. Außerdem können durch zusätzliche Elektroden im Kanalsystem elektroosmotische Flüsse erzeugt werden.

Die nichtleitenden Substrate bestehen bevorzugt aus einem Material ausgewählt aus der Reihe: Glas, SiO₂, Kunststoffen, z.B. aus Polyethylenterephtalat, Polycarbonat, Polystyrol.

Für die Elektroden kommen Metalle wie Au, Pl, Ag, Ti, Halbleiter wie z.B. Si, insbesondere dotiertes Si, Metalloxide insbesondere Indium-Zinn-Oxid (ITO) oder leitfähige Polymere wie Polyaniline, Polythiophene insbesondere Polyethylendioxythiophen, Polyphenylene, Polyphenylenvinylen, Polythiophenvinylen, Polypyrrole in Frage.

Gegenstand der Erfindung ist ferner die Verwendung der Vorrichtung als Nukleinsäure-Array, Peptid-Array oder als Protein-Array.

Mit dem beschriebenen Verfahren und Vorrichtung wird in Bezug auf den nächstliegenden Stand der Technik (WO 02/02810) ein einfacherer Elektrodenaufbau ermöglicht, da eine metallische Überbrückung von eng benachbarten Elektroden nicht erforderlich ist und auch nur eine gemeinsame Gegenelektrode benötigt wird. In Bezug auf den nächstliegenden Stand der Technik (Cai et al., Analytica Chimica Acta 469, 165-172 (2002)) lässt das beschriebene Verfahren höhere Empfindlichkeiten durch die Ausbildung der elektrisch leitfähigen Schicht auf der Messfläche erwarten. Darüber hinaus kann durch die während der Verstärkung erfolgende Messung der Leitfähigkeit der Verstärkungsprozess beurteilt werden und gegebenenfalls zeitsparend abgebrochen werden und macht auch zusätzliche Schritte wie z.B. Waschschritte überflüssig.

Die Erfindung wird nachstehend anhand der Figuren beispielhaft näher erläutert.

Es zeigen
- Fig. 1: Ein Schema der Messvorrichtung
- Fig. 2: das Widerstands-Zeit-Diagramm einer DNA-Hybridisierungsreaktion mit Kontrollhybridisierungsreaktion
- Fig. 3: einen Sensor mit zusätzlicher leitfähigen Fläche
- Fig. 4: die vertikale Anordnung von Elektroden-Isolatorschichtfolgen

### Beispiele

### Beispiel 1

### Methode und Vorrichtung zur Detektion von DNA auf einem Polymer-Chip

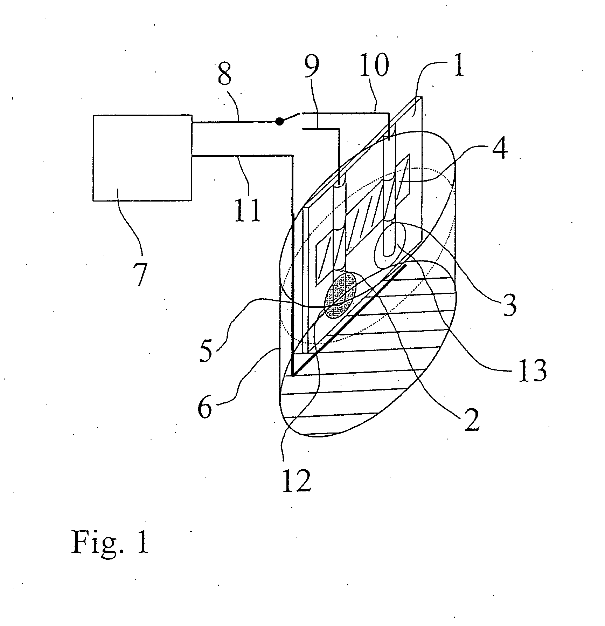

Der Sensor der Messvorrichtung besteht im wesentlichen aus einem PolyethylenterephtalatSubstrat 1 mit aufgedruckten Kohleelektroden 2 und 3, s. Fig. 1. Über die Elektroden 2 und 3 ist ein isolierende Polymerschicht 4 aufgebracht, die sich zwischen den Anschlussstellen des Sensors und den Bereich der Messelektroden befindet. Die Sensoren wurden von der Firma Molecular Circuitry, West Conshohocken, PA, USA bezogen. Als Gegenelektrode dient ein Ag-Draht 5, der ca. 1 mm entfernt vom Sensor in einer Petrischale 6 montiert ist. Als Messgerät 7 dient ein Keithley 2000 Multimeter, das im Widerstand-Modus, Messbereich 10 MOhm, betrieben wird. Die Eingänge des Messgeräts können wahlweise über die Zuleitungen 8, 9, 10 mit den Elektroden 2 oder 3 und dem Ag-Draht 5 über die Zuleitung 11 verbunden werden.

Zur Reinigung wurden die Sensoren im ersten Reinigungsschritt 30 min in Ethanol eingelegt und anschließend in ddH₂0 gespült. Dieser Reinigungsschritt wurde einmal wiederholt. Im zweiten Reinigungsschritt wurden die Sensoren 15 min in 2 % Alconox-Lösung eingelegt und anschließend mit ddH₂0 gespült. Auch dieser Reinigungsschritt wurde einmal wiederholt.

Die Sensoren wurden in eine wässrige Poly(Phenylalanin)-Lysin (Sigma) (0,1mg/ml)-Lösung bei 2M NaCl und 50 mM KH₂PO₄ (pH 7.1) 1 h bei RT inkubiert, um eine Polymerschicht auf den Sensor aufzuziehen.

Auf den Sensoren wurden zwei unterschiedliche Bereiche von Erkennungselementen immobilisiert: Erkennungs-DNA-Positiv (5'-Amino-GTCCCCTACGGACA.AGGCGCGT-3') auf Positiv-Bereich 12 und Erkennungs-DNA-Negativ (5'-Amino--TTTTTCGCGCCTTGTCCGTAGGGGACT-3') auf Negativ-Bereich 13. Hiermit werden für einen Analyten Positiv- und Negativkontrolle simultan in einem Assay bei Zugabe nur eines Positiv-Analytens ermöglicht.

Erkennungs-DNAs wurden in Phosphatpuffer pH 7,2 gelöst und mit 0,1 M Bis-sulfo-succinimidylsuberat (BS3, Hersteller Fa. Pierce) 10 min bei RT inkubiert. Durch Verdünnung mit Phosphatpuffer wurde die Reaktion abgebrochen. Die Reinigung der Erkennungs-DNA erfolgte durch Chromatographie auf einer NAP-10-Säule (Hersteller Fa. Pharmacia). Die gereinigte Erkennungs-DNA wurde in Volumina von z.B. 20 µl auf die Oberflächen des Sensors aufgebracht und über Nacht bei Raumtemperatur inkubiert. Die resultierenden DNA-Chips wurden mit 1 % wässriger Ammoniumhydroxid-Lösung sowie ddH₂O gewaschen. Zur Blockierung der nicht umgesetzten Aminogruppen auf der Chipoberfläche wurde der Sensor 4 h mit einer aktivierten Carboxymethyl-Dextran-Lösung inkubiert. Die Lösung wurde wie folgt hergestellt: Teill: 20 mg/ml Carboxymethyl-Dextran in ddH₂O und Teil2: 0,2 mmol/ml EDC + 0,2 mmol/ml NHS in ddH₂O lösen. Lösung Teil1 + Lösung Teil 2 im Verhältnis 1:1 mischen und 20 min reagieren lassen. Die Sensoren wurden anschließend 1 h in ddH₂O inkubiert.

Auf den mit Erkennungs-DNAs beschichteten Sensoroberflächen 12 und 13 wurden DNA-Hybridisierungsreaktionen mit einer Analyt-DNA-Probe der Sequenz 5'-Biotin-TTTTTCGCGCCTTGTCCGTAGGGGACT-3'durchgeführt. Es wurde eine 10⁻⁸ M Lösung der DNA in Tris-Puffer pH 8, 1M NaCl, 0,005 % SDS auf dem Sensor in einem Volumen von 20 µl über Nacht bei 56°C inkubiert. Anschließend wurde mit Hybridisierungspuffer gewaschen, um nicht hybridisierte DNA von der Chipoberfläche zu entfernen. Die hybridisierte Target-DNA wurden 4 h bei RT mit einer Lösung von Streptavidin-Gold (Durchmesser der Goldpartikel 10 nm, Sigma) inkubiert. Die Sensoren wurde mit Hybridisierungspuffer gewaschen, bei dem 1 M NaCl durch 1 M NaNO₃ ausgetauscht wurde, und anschließend bei RT getrocknet. 50 µl der Verstärkungslösung (1:1 Mischung aus (4,8 µl 1 M AgNO₃ auf 0,2 ml 0,3 M Citratpuffer) und (61 mg Hydrochinon auf 5 ml 0,3 M Citratpuffer) wurde auf den Sensor für 1. min gegeben. Anschließend wurden die Sensoren 2x mit ddH₂O gespült und getrocknet.

Für die Online-Messung wurde der Sensor in ca. 6 ml der Verstärkungslösung (1:1 1 Mischung aus (4,8 µl 1 M AgNO₃ auf 0,2 ml 0,3 M Citratpuffer) und (61 mg Hydrochinon auf 5 ml 0,3 M Citratpuffer) eingetaucht. Im Abstand von 20 s wurde alternierend das Widerstands-Messgerät 7 für ca. 10 s zwischen die Elektroden 9, 10 und der Gegenelektrode 11 gelegt und der Widerstand gemessen.

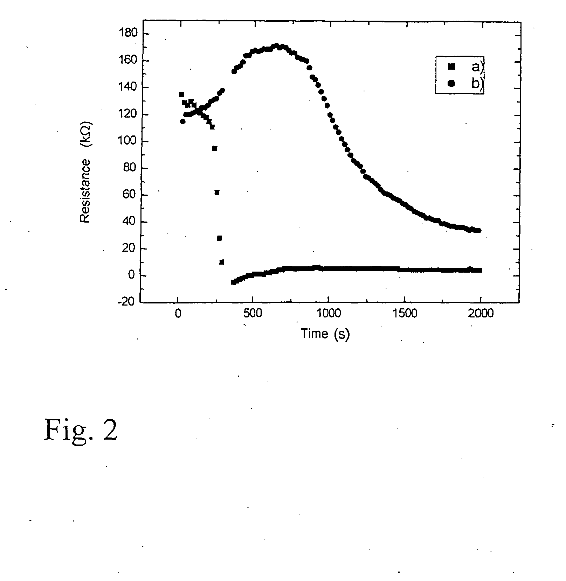

Fig. 2 zeigt den Widerstand als Funktion der Zeit während des Ag-Verstärkungsprozesses für a) die Positivreaktion der Analyt-DNA mit der Erkennungs-DNA-Positiv und b) der Kontrollhybridisierungsreaktion von Analyt-DNA mit der Erkennungs-DNA-Negativ. Für die positive Reaktion lässt sich eine signifikante sprunghafte Erniedrigung des Widerstands bei t = 220 s detektieren. Im Vergleich hierzu erfolgt der Widerstandsabfall bei der negativen Reaktion deutlich später und nicht so steil.

### Beispiel 2

### Methode und Vorrichtung zur Detektion von DNA mit zusätzlicher leitfähiger Schicht

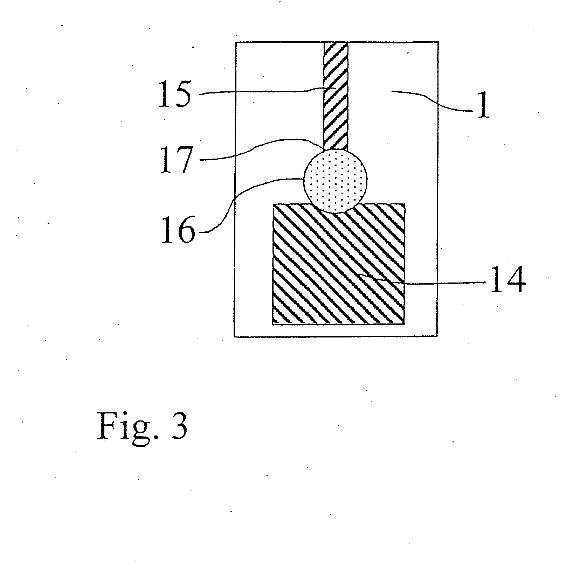

In Fig. 3 ist eine weitere Ausführungsform realisiert, die eine durch die Erkennungsreaktion bewirkten Anbindung einer leitfähigen Fläche 14 aus Au an die Kohle-Messelektrode 15 ermöglicht. Leitfähige Fläche und Elektrode sind wie in Beispiel 1 auf einem isolierendes Substrat 1 aus Polyethylenterephtalat aufgebracht. Die Messfläche wird durch den Immobilisierungsbereich 16 definiert und deckt daher sowohl das Ende 17 der Elektrode 15 definiert die Messfläche. Die Präparation dieses Sensors, Durchführung einer Hybridisierungsreaktion und die Ag-Verstärkung mit simultaner Widerstandsmessung wird analog zu Beispiel 1 durchgeführt.

### Beispiel 3

### Detektion von DNA mit einer Vorrichtung mit übereinander angeordneter Elektroden-Isolatorschichtfolge

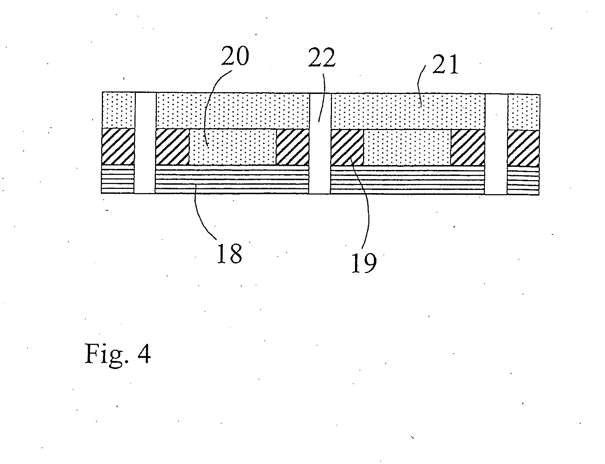

Eine alternative Ausführung für einen erfindungsgemäßen Sensor ist eine übereinander angeordnete Elektroden-Isolatorschichtfolge nach Fig. 4. Auf ein Glassubstrat 18 wird eine Au-Schicht abgeschieden. Mittels Fotolithografie wird diese Schicht in einzelne Bereiche 19 vereinzelt. Die Zwischenräume 20 werden anschließend mit SiO₂ über Plasmaabscheidungsverfahren aufgefüllt. Auf diese Schicht wird eine SiO₂-Deckschicht 21 durch die gleiche Methode aufgebracht. Durch Ionenstrahlätzen wird ein Mikrokanal 22 einer Weite von z.B. 20 µm durch die Schichtstruktur realisiert. Die Immobilisierung von aminofunktionalisierter DNA wird über bekannte Silanisierungsverfahren wie z.B. mit Aminopropyltriethoxysilan (APTES) an der SiO₂-Innenseite dieses Mikrokanals und einem bifunktionellen Linker BS³ aus Beispiel 1 erreicht. In diesem Beispiel werden auf den Messflächen der einzelnen Kanäle jeweils verschiedene Erkennungs-DNAs für die simultane Detektion unterschiedlicher Analyten immobilisiert. Die Durchführung des Assays sowie Ag-Verstärkung mit simultaner Widerstandsmessung findet analog zu Beispiel 1 statt, wobei lediglich die Reaktionslösungen mit einem spritzenbetriebenen Schlauchsystem durch die Öffnungen der Vorrichtung gespült werden. Die elektrischen Widerstände der einzelnen Bereiche 19 gegen eine einzige Gegenelektrode im Reaktionsgefäß werden nacheinander oder simultan gemessen.

## Patentansprüche

1. Verfahren zum Nachweis eines oder mehrerer Analyten durch eine Erkennungsreaktion, unter Verwendung
- einer Vorrichtung mit einem mit Elektrolyten gefüllten Elektrolytraum aufweisend
- mindestens eine Messelektrode mit gegebenenfalls einem, an die Messelektrode angrenzenden isolierenden Bereich (Isolierschicht)
- Erkennungsmolekülen, die auf der Messelektrode und/oder auf dem an die Messelektrode/n angrenzenden isolierenden Bereich immobilisiert sind und einer Gegenelektrode,
A) in Kontaktbringen von mit elektrisch aktiven Markierungseinheiten markierten Analyten mit den Erkennungsmolekülen, wobei die elektrisch aktiven Markierungseinheiten entweder vor dem Kontakt der Analyten mit Erkennungsmolekülen an die Analyten gebunden worden sind oder nach dem Kontakt der Analyten mit der biofunktionellen Oberfläche an die Analyten gebunden werden,
B) Applikation einer reaktiven Verstärkungslösung in den Elektrolytraum zur Verstärkung/Erzeugung der elektrischen Leitfähigkeit der elektrisch aktiven Markierungseinheiten,
C) Messung der elektrischen Leitfähigkeit bzw. des Widerstands zwischen der oder den Messelektrode/n und der Gegenelektrode in Abhängigkeit von der Entwicklungszeit,
D) Analyse des zeitlichen Verlaufs der Leitfähigkeit bzw. des Widerstands nach C).

2. Verfahren nach einem der Anspruch 1, **dadurch gekennzeichnet, dass** die Konzentrationsangabe in einem quantitativen Assay bzw. der grundsätzliche Nachweis in einem qualitativen Assay über die Zeitdauer der Reaktion B) bis zum Erreichen eines signifikanten Gradienten im zeitlichen Verlaufs von Widerstand oder Leitfähigkeit mit Zeitdauer der Reaktion B) einer Referenzprobe verglichen und zur quantitativen oder qualitativen Analyse des Analyten verwendet wird.

3. Verfahren nach einem der Ansprüche 1 bis 2, **dadurch gekennzeichnet, dass** die Erkennungsmoleküle über eine biofunktionelle Oberflächenbeschichtung auf der Messelektrode und/oder der angrenzenden Isolierschicht angebracht sind.

4. Verfahren nach einem der Ansprüche 1 bis 3, **dadurch gekennzeichnet, dass** in Schritt A) eine quantitativ vorbestimmte Menge eines bekannten Analyten, der mit Markierungseinheiten versehen ist, gemischt wird mit der Probe eines unmarkierten bekannten Analyten und aus einem Vergleich der Analyse D) dieses Mischsystems mit der Analyse des reinen bekannten markierten Analyten die Konzentration des unmarkierten Analyten bestimmt wird.

5. Verfahren nach einem der Ansprüche 1 bis 4, **dadurch gekennzeichnet, dass** ein Analytmolekül mit einem oder mehreren elektrisch aktiven Markierungseinheiten markiert wird.

6. Verfahren nach einem der Ansprüche 1 bis 5, **dadurch gekennzeichnet, dass** die elektrisch aktiven Markierungseinheiten nichtleitfähige Partikel auf Basis von Polymeren mit leitfähiger Beschichtung, bevorzugt nichtleitfähige Partikel mit metallischer Beschichtung sind.

7. Verfahren nach einem der Ansprüche 1 bis 5, **dadurch gekennzeichnet, dass** die elektrisch aktiven Markierungseinheiten auf leitfähigen Polymeren basieren.

8. Verfahren nach einem der Ansprüche 1 bis 7, **dadurch gekennzeichnet, dass** eine Vielzahl von Erkennungsreaktionen mit den' Schritten A) bis D) parallel simultan auf einer Sensorvorrichtung durchgeführt werden, indem die Messvorrichtung mit einer Vielzahl von Messflächen versehen werden, auf denen jeweils gleiche oder unterschiedliche Erkennungselemente angebracht sind.

9. Vorrichtung zum Nachweis eines oder mehrerer Analyten durch eine Erkennungsreaktion, insbesondere nach einem Verfahren gemäß einem der Ansprüche 1 bis 8, wenigstens umfassend
- mindestens eine Messelektrode und mindestens einen angrenzenden isolierenden Bereich (Isolierschicht)
- Erkennungsmoleküle, die auf dem an die Elektrode/n angrenzenden isolierenden Bereich und optional auf der Elektrode/n immobilisiert sind,
- eine Gegenelektrode
- Analyten, die mit elektrisch aktiven Markierungseinheiten markiert sind
- gegebenenfalls eine Verstärkungslösung und ein Messgerät zur zeitaufgelösten Erfassung der Leitfähigkeit bzw. des Widerstands zwischen der oder den Messelektrode/n und Gegenelektrode während des Verstärkungsprozesses.

10. Vorrichtung nach Anspruch 9, **dadurch gekennzeichnet, dass** die Gegenelektrode bzw. die Referenz- und Gegenelektrode zusammen mit der Messelektrode auf einem gemeinsamen Substrat angebracht sind.

11. Verwendung der Vorrichtung nach einem der Ansprüche 9 bis 10 als DNA-Array oder als Protein-Array.
